# EUROPEAN PATENT SPECIFICATION

(11) **EP 1 394 132 B1**
(45) Date of publication and mention of the grant of the patent: **12.09.2012**
(21) Application number: 03017236.5
(22) Date of filing: 30.07.2003
(51) Int. Cl.: C04B 12/02, A61L 27/12, A61K 6/06

(54) **A new calcium phosphate cement composition and a method for the preparation thereof**
Neue Calciumphosphatzementzusammensetzung und ihr Herstellungsverfahren
Nouvelle composition de ciment de phosphate de calcium et son procédé de préparation

(30) Priority: 27.08.2002 EP 02019214
(43) Date of publication of application: 03.03.2004
(73) Proprietor: Biomet Deutschland GmbH, 14167 Berlin (DE)
(72) Inventor: Tas, Ahmed Cueneyt, Dr., Central, SC 29634 (US)
(74) Representative: Dey, Michael

(56) References cited:
- EP-A- 1 172 076
- EP-A1- 1 023 032
- WO-A-95/08319
- GB-A- 2 248 232
- US-A- 6 027 742
- US-A1- 2002 073 894
- DRIESSENS F C M ET AL: "Effect of temperature and immersion on the setting of some calcium phosphatecements" JOURNAL OF MATERIALS SCIENCE. MATERIALS IN MEDICINE, CHAPMAN AND HALL, LONDON, GB, vol. 11, 2000, pages 453-457, XP008083523 ISSN: 0957-4530
- CUNEYT TAS A ET AL: "An investigation of the chemical synthesis and high-temperature sintering behaviour of calcium hydroxyapatite (HA) and tricalcium phosphate (TCP) bioceramics" JOURNAL OF MATERIALS SCIENCE. MATERIALS IN MEDICINE, CHAPMAN AND HALL, LONDON, GB, vol. 8, no. 2, February 1997 (1997-02), pages 91-96, XP008083739 ISSN: 0957-4530
- MIRTCHI A A ET AL: "CALCIUM PHOSPHATE CEMENTS: STUDY OF THE -TRICALCIUM PHOSPHATE - DICALCIUM PHOSPHATE - CALCITE CEMENTS" BIOMATERIALS, ELSEVIER SCIENCE PUBLISHERS BV., BARKING, GB, vol. 11, no. 2, 1 March 1990 (1990-03-01), pages 83-88, XP000100849 ISSN: 0142-9612
- DRIESSENS F C M ET AL: "EFFECTIVE FORMULATIONS FOR THE PREPARATION OF CALCIUM PHOSPHATE BONE CEMENTS" JOURNAL OF MATERIALS SCIENCE. MATERIALS IN MEDICINE, CHAPMAN AND HALL, LONDON, GB, vol. 5, no. 3, 1994, pages 164-170, XP000929539 ISSN: 0957-4530
- AKAO, M. ET. AL.: "Dense polycrystalline beta-tricalcium phosphate for prosthetic applications" J. MATER. SCI., vol. 17, 1982, pages 343-346, XP008083737
- JARCHO M ET AL: "SYNTHESIS AND FABRICATION OF BETA-TRICALCIUM PHOSPHATE (WHITLOCKITE) CERAMICS FOR POTENTIAL PROSTHETIC APPLICATIONS" JOURNAL OF MATERIALS SCIENCE, CHAPMAN AND HALL LTD, GB, vol. 14, no. 1, January 1979 (1979-01), pages 142-150, XP008033115 ISSN: 0022-2461
- JARCHO ET AL: "Hydroxylapatite synthesis and characterization in dense polycrystalline form" JOURNAL OF MATERIALS SCIENCE, CHAPMAN AND HALL LTD, GB, vol. 11, no. 11, November 1976 (1976-11), pages 2027-2035, XP008033116 ISSN: 0022-2461
- TAS A C: "Molten salt synthesis of calcium hydroxyapatite whiskers" JOURNAL OF THE AMERICAN CERAMIC SOCIETY, BLACKWELL PUBLISHING, MALDEN, MA, US, vol. 84, 2001, pages 295-300, XP008083547 ISSN: 0002-7820

## Description

The invention describes a new calcium phosphate cement powder, whose composition can best be described over the Ca/P molar ratio range of 1.35 to 1.40, optimally 1.39, and whose two components were prepared by wet chemical synthesis procedures. One component is chemically-synthesized, bi-phasic alpha-TCP (Ca₃(PO₄)₂, 95 wt%) + HA (Ca₁₀(PO₄)₆(OH)₂, 5 wt%) powder, while the second component is again a chemically-synthesized, single-phase DCPD (CaHPO₄·2H₂O) powder. A setting solution (3 wt% Na₂HPO₄·2H₂O dissolved in distilled water) is used to form a self-setting calcium phosphate cement from the powder mixture. This cement can be used as bone filler or bone substitute in applications, which require higher rates of resorption.

### Background of the Invention

Calcium phosphate-based cements (**a.)** H. Monma and T. Kanazawa, "Wet-Process Formation of Non-stoichiometric Hydroxyapatite from Tricalcium Phosphate," Yogyo Kyokaishi, 86, 73-76, 1978, **b.)** W. E. Brown and L. C. Chow, "A New Calcium Phosphate Water Setting Cement"; pp. 352-77 in Cements Research Progress-1986, Edited by P. W. Brown. American Ceramic Society, Westerville, Ohio, 1987, **c.)** A. A: Mirtchi, J. Lemaitre, and E. Munting, "Calcium Phosphate Cements: Study of the beta-tricalcium Phosphate-Dicalcium Phosphate-Calcite Cements," Biomaterials, 11, 83-88, 1990, **d.)** F. C. M. Driessens, J. A. Planell, et al., Bioceramics, 10, 279-82, 1997, **e.)** K. S. TenHuisen and P. W. Brown, "Formation of Calcium-Deficient Hydroxyapatite from alpha-Ca3(PO4)2," Biomaterials, 19, 2209-17, 1998.) are conventionally prepared by mixing calcium phosphate powders of a special composition and a kneading liquid, such as distilled water, for example, in a mortar to obtain kneaded cement which may then be filled into or applied to a defective portion of bone (or tooth) using a syringe or spatula or hand and then allowed to cure.

Calcium phosphate-based cements are usually desired to be almost identical with the chemical composition of the inorganic component of bones or teeth, which is carbonated, deficient or stoichiometric "calcium hydroxyapatite." However, in recent years with an increase in the number of animal studies performed with such materials, it is becoming more and more evident that the calcium hydroxyapatite bioceramic, when prepared synthetically or even when taken from bovine sources in highly porous forms (i.e., granules or blocks), has very low bioresorbability (M. T. Mushipe, P. A. Revell, and J. C. Shelton, "Cancellous Bone Repair using Bovine Trabecular Bone Matrix Particulates," Biomaterials, 23, 365-370, 2002), and moreover, if it is stoichiometric (i.e., its Ca/P molar ratio being equal to 1.67) it almost doesn't take part in the bone remodelling processes which were initiated and performed by the bone cells *in vivo.*

Calcium phosphate-based cements when they are prepared by using calcium phosphate powder formulations which have a Ca/P molar ratio values higher than 1.50 (e.g., F. C. M. Driessens, M. G. Boltong, E. A. P. De Maeyer, R. M. H. Verbeeck, and R. Wenz, " Effect of temperature and immersion on the setting of some calcium phosphate cements," J. Mater. Sci. Mater. Medic., 11, 453-57, 2000) do also show reduced levels of resorbability (as compared to calcium phosphate cements (e.g., U.S. Pat. No. 6,117,456) of lower Ca/P molar ratios) when implanted *in vivo.*

However, the Ca/P molar ratio of calcium phosphate-based cements do not alone dictate the extent of *in vivo* resorbability of these. Together with the appropriate adjustment of the overall Ca/P ratio, the proper choice of the calcium phosphate compounds (in an order of decreasing *in vitro* solubility at neutral pH values: TTCP (Ca₄(PO₄)₂O), alpha-TCP (Ca₃(PO₄)₂), MCPM (Ca(H₂PO₄)₂·H₂O), beta-TCP, Ca₂P₂O₇, DCPD (CaHPO₄·2H₂O), DCPA (CaHPO₄), or HA (Ca₁₀(PO₄)₆(OH)₂)) to be used in the design of cements becomes the crucial factor in tailoring the resorbability of a new cement.

Homogenous powders of hydroxyapatite (HA), tricalcium phosphate (TCP) and mixtures thereof can be synthesized by chemical precipitation from aqueous solution of calcium nitrate and ammonium phosphate salts (A. Cüneyt Tas, F. Korkusuz, M. Timucin, N. Akkas "An investigation of the chemical synthesis and high-temperature sintering behaviour of calcium hydroxyapatite (HA) and tricalcium phosphate (TCP) bioceramics" J. Material Science: Materials in Medicine, 8, 91-96, 1997).

In selecting the calcium phosphate compounds (either from the binary system of CaO-P₂O₅ or from the ternary system of CaO-P₂O₅-H₂O) to form a cement powder out of those, utmost care and priority must also be given to the *in vitro*/*in vivo* solubility (and the rate of hydrolysis of those in media similar to human plasma) of the candidates under consideration.

Calcium phosphate cements for living bodies have an advantage that most of them transform into a bioactive hydroxyapatite (also known as "apatitic tricalcium phosphate," Ca₉(HPO₄)(PO₄)₅OH) upon hardening, and hence result in a hardened cement having excellent bioaffinity. Many of the already known calcium phosphate cements for living bodies comprise tetracalcium phosphate (TTCP, Ca₄(PO₄)₂O) as the main component. For example, U.S. Pat. No. 4,612,053 and EP No. 1172076 disclose cements comprising tetracalcium phosphate and dicalcium phosphate anhydrous (DCPA, CaHPO₄) as the main components, whereas the US Pat. No. 5,525,148 describes the preparation of a series of calcium phosphate cements which do not contain any TTCP. It is also known that the hardening properties (i.e., setting times (typically measured in the dry state) and final compressive strengths achieved following immersion in pseudo or real physiological fluids) of these calcium phosphate cements widely vary also depending on the amount of liquid employed in the step of kneading. That is, the hardening time is shortened while the strength of the hardened body is elevated with a decrease in the kneading liquid employed.

The most popular TTCP-containing cement (whose secondary component being the acidic calcium phosphate, MCPM: Ca(H₂PO₄)₂·H₂O) is known under the commercial name of "Norian SRS," and it has a compressive strength in the vicinity of 40 MPa, according to its manufacturer (Norian Corporation). This cement has a Ca/P molar ratio slightly greater than 1.50. Its *in vivo* resorbability still requires the disclosure of animal and clinical tests from independent sources.

U.S. 6,117,456 discloses the preparation of a highly resorbable (complete *in vivo* resorption in less than a year) cement of the name alpha-BSM (which is marketed in Europe (by Biomet-Merck) under the name of "BIOBON^{®}"). This cement consists of two powder components, (i) poorly crystalline calcium phosphate (major phase), and (ii) well-crystallized DCPD (Brushite, CaHPO₄·2H₂O). BIOBON^{®} has a Ca/P molar ratio less than 1.50. Although it is major, poorly crystalline calcium phosphate component reacts quite rapidly (started within the first 24 hours, and continues with the passage of time) to form apatitic tricalcium phosphate (Ca₉(HPO₄)(PO₄)₅OH), the full resorption of the crystalline component takes significantly longer to take place. BIOBON^{®} (or alpha-BSM), which is kneaded with a simple saline solution to form its paste, suffers from extremely low compressive strength values (in the vicinity of 10 to 15 MPa) upon full setting, and this severely limits its usage mainly to "non-load-bearing" places and applications.

U.S. 5,152,836 describes a calcium phosphate cement (again with a Ca/P molar ratio slightly greater than 1.50) composed of alpha-TCP (75 wt%), TTCP (18 wt%), DCPD (5 wt%), HA (2 wt%), kneaded into a paste with a relatively concentrated aqueous solution of chondroitin sulphate and sodium succinate. This cement has been in the market under the commercial name of BIOPEX^{®} (Mitsubishi Material Co.). It is claimed to achieve a compressive strength of 60 to 90 MPa. Little is known about its resorbability, but it is claimed by its manufacturer to resorb quite fast (around 50% in few weeks).

A cement composition containing 64 wt% α-TCP, 9 wt% PHA (precipitated hydroxyapatite) and 27 wt% DCP (Dicalcium phosphate CaHPO₄) is known under the name Biocement F. Biocement F, however, is characterized by a short initial setting time making at temperatures above 27°C making it thus difficult to use in warmer settings (F.C.M. Driessens, M.G. Boltong, .E.a.P. De Maeyer, R.M.H. Verbeeck "effect of temperature and immersion on the setting of some calcium phosphate cements" J.Materials Science:Materials in Medicine, 11, 453-457, 2000).

The newest calcium phosphate cement commercially available on the market is known as CALCIBON^{®} (produced and marketed by Biomet-Merck) with a Ca/P molar ratio of 1.55, and it consists of a mixture of alpha-TCP (58-60 wt%), DCPA (26-27 wt%), CaCO₃ (12-13 wt%), and HA (2%). It has a compressive strength over the range of 50-60 MPa, and in the bulk form (i.e., without any significant macroporosity) it is not as fast-resorbable as BIOBON® . High compressive strength calcium phosphate cements are nevertheless still suitable for the repair of bone cavities or defects in load-bearing places of the living bodies.

Alpha-TCP, alone, is known to easily hydrolyze *in vitro* or *in vivo* directly into calcium-deficient hydroxyapatite (K. S. TenHuisen and P. W. Brown, "Formation of Calcium-Deficient Hydroxyapatite from alpha-Ca3(PO4)2," Biomaterials, 19, 2209-17, 1998), and the Ca/P molar ratios in a wide family of "calcium-deficient hyroxyapatites" can take values over the range of 1.3 to 1.65. When these values are in excess of 1.50, and when they become progressively closer to that of stoichiometric hydroxyapatite (1.67), the resorbability of the implants is observed to decrease. On the other hand, if the formed calcium-deficient hydroxyapatites (as a result of the setting reaction) also contain alkali elements like Na and K, then the resorbability of the cements would also increase (F. C. M. Driessens, M. G. Boltong, E. A. P. de Maeyer, R. Wenz, B. Nies, and J. A. Planell, "The Ca/P Range of Nanoapatitic Calcium Phosphate Cements," Biomaterials, 23, 4011-17, 2002). The intentional doping of crystallographic Ca-sites in the newly forming calcium-deficient hydroxyapatite microstructure (which is typically imaged in electron microscope micrographs with microflakes or microneedles forming on the alpha-TCP grains) with such alkali elements leads to the generation of vacancies, and carbonate ion (CO₃²⁻) substitutions in the hydroxyl sites and the phosphate ion sites, respectively. It also needs to be remembered hereby that the human bones contain around 1.6 wt% Na and K ions.

The primary powder components (i.e., alpha-TCP and TTCP) for almost all of the commercially available calcium phosphate cements, with the only exception of BIOBON^{®}, have been prepared by solid-state reactive firing (SSRF) at high temperatures (in excess of 1350°C). The use of such high temperatures during production inescapably lead to hard, sintered products with grain sizes mostly in excess of 80 to 100 µm, and therefore those components are needed to be grinded with high-energy mills, first, into a fine powder before their use in the cement formulations. Fine powders (less than 30 µm) are strictly required in the calcium phosphate cement formulations in order to achieve higher rates of *in vivo* bioreactivity and biointegration with the ingrowing bone into the repair site. SSRF practices and the follow-up grinding operations naturally increase the costs of manufacturing of such cements.

### Summary of the Invention

An object of the present invention is to provide a new calcium phosphate cement, which avoids the above-mentioned disadvantages from the prior art and having a Ca/P molar ratio significantly lower than 1.50, and whose major component being α-Ca₃(PO₄)₂ (i.e., α-TCP), a minor component being the high aqueous solubility calcium phosphate compound Brushite (DCPD: CaHPO₄x2H₂O), and to contain a small amount of hydroxyapatite to serve as a seed to accelerate the formation of calcium-deficient hydroxyapatite.

Another object of the invention is to provide a method of preparing an α-TCP-based calcium phosphate cement, whose entire constituents are produced by wet-chemical synthesis routes, which at the same time facilitate easier alkali element (Na and K) doping into the cement body, and thereby eliminating the cost-increasing processing steps, such as the use of temperatures in excess of 1200° C on the production floor, and tedious high-energy grinding operations for decreasing the particle sizes.

Upon further study of the specification and appended claims, further features and advantages of this invention will become apparent to those skilled in the art.

These and other objects are achieved by a method of preparing a calcium phosphate cement composition, characterized in that the method comprises the steps of:
a) adding a preheated Ca(NO₃)₂ x 4H₂O solution to a (NH₄)₂HPO₄ solution under stirring followed by addition of concentrated NH₄OH solution and subsequently calcining at about 1200° C of 95 wt% β-type calcium tertiary phosphate and 5 wt% hydroxyapatite to form biphasic **powder A** consisting of 95 wt% α-type calcium tertiary phosphate and 5 wt% hydroxyapatite.
b) adding a Na₂HPO₄x₂H₂O solution to a KH₂PO₄ solution under stirring followed by adding of Ca(NO₃)₂x4H₂O to form single-phase **powder B** (CaHPO₄x2H₂O).
c) mixing of **powder A** with **powder B** in a mill and
d) subsequently adding a setting solution (Na₂HPO₄x2H₂O) to form the cement paste with an overall molar ratio of Ca/P of 1.35 to 1.40.

The calcium phosphate cement powder of this invention is formed by physically mixing two powders together. These powders are (a) Powder A: a bi-phasic mixture of alpha-TCP (α-Ca₃(PO₄)₂, 95 wt%) + HA (Ca₁₀(PO₄)₆(OH)₂, 5 wt%), and (b) Powder B: DCPD (CaHPO₄·2H₂O). These powders are prepared by wet-chemical synthesis procedures, whose details are given in the working examples below. Cement powder is obtained by blending 70 to 80 wt% powder A and 20 to 30 wt% powder B in a mill with one another. Preferred is a mixing ratio of 75 : 25 by weight.

The preferred setting solution to cause the initiation of the setting reaction, is an aqueous 3 wt% Na₂HPO₄·2H₂O solution. It is also observed that increasing the concentration of this solution to 4 wt% decreased the hardening time, while decreasing it (to 2 wt%) extended the hardening time beyond 30 minutes.

The preferred "liquid-to-powder" (i.e., L/P) ratio for this cement was in the range of 0.40 to 0.45 mL of solution per gram of cement powder. The most preferable value was 0.43 mL.

When the Ca/P molar ratio is adjusted (by changing the mixing ratios of Powder A and Powder B) between 1.33 and 1.43, it was also observed that the setting reaction takes place. Starting from the lower end (1.33) of this Ca/P ratio range, in going to its upper end (1.43), compressive strength has the tendency to increase (from 34 to 39 MPa).

Calcined powders are lightly ground to obtain a fine powder with particles less than 40 µm.

The invention is described in detail below in terms of the following working examples.

In the foregoing and in the following examples, all temperatures are set forth uncorrected in degrees Celsius; and, unless otherwise indicated, all parts and percentages are by weight.

### EXAMPLE 1

### Synthesis of bi-phasic alpha-TCP+HA powders (Powder A):

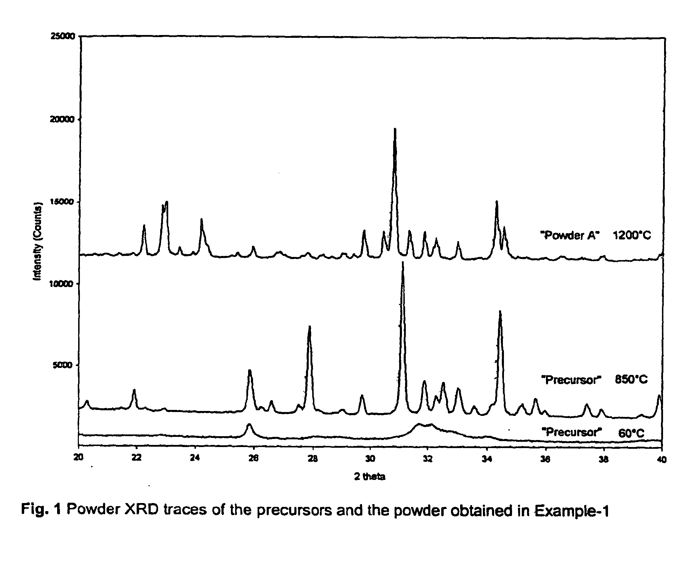

51.53 g of (NH₄)₂HPO₄ is dissolved in a glass beaker in 650 mL distilled H₂O, preheated to 37°C, to form a clear solution (Solution A). In a separate glass beaker 139.35 g of Ca(NO₃)₂·4H₂O is dissolved in 1000 mL of H₂O, preheated to 37°C, to form solution B. Solution B is slowly (in 5 minutes) added into solution A under constant stirring. The temperature of the opaque solution is maintained at 37°C. The nominal Ca/P molar ratio in this solution is 1.512. A 33 mL aliquot of concentrated (i.e., 25 vol%) NK₄OH was then added at once to the milky solution, and stirred for 2 hours at 37°C. Formed precipitates are then filtered out of the mother liquor, washed with 2 liters of distilled water, followed by drying in an air atmosphere at 60°C for 24 hours. The dried powders are later calcined in an inert Al₂O₃ bowl at 850°C for 12 hours in an air atmosphere. Formed powders are found to consist of 95 wt% beta-TCP and 5 wt% HA. These sub-micron particulated powders are then converted to 95 wt% alpha-TCP + 5 wt% HA by calcining at 1200°C. followed by quenching to room temperature. Calcination is performed as follows: 95 wt% beta-TCP + 5 wt% HA powders are heated (in an Al₂O₃ bowl) from room temperature to 1200°C in 4 hours, soaked at 1200°C for 3.5 hours, followed by quenching (in the furnace) from 1200°C to 1000°C in 10 minutes, subsequent cooling from 1000° to 500°C in 1 h, and final cooling to RT from 500°C being achieved in 3 hours. Calcined powders are lightly ground to obtain a fine powder with particles less than 40 µm. (see Fig. 1)

Figure 1 shows the powder X-ray diffraction (XRD) data for the two precursors of powder A (obtained at 60° and 850°C), and the data for the Powder A itself (after 1200°c calcination) in one diagram.

### EXAMPLE 2

### Synthesis of Brushite (DCPD: CaHPO₄·2H₂O) powders (Powder B):

2.0636 g of KH₂PO₄ are dissolved in a glass beaker containing 1750 mL of distilled water at room temperature to prepare a clear solution. 7.5324 g of Na₂HPO₄·2H₂O is then added into this solution and mixed for 15 minutes. The pH value of the resultant solution is measured to be 7.4. 27.59 g of Ca(NO₃)₂·4H₂O (in powder form) is then added at once into the solution B, and mixed at room temperature for 80 minutes. Formed precipitates are then filtered out of the mother solution, washed with 2 liters of distilled H₂O, and dried at 60°C for 24 hours. High crystallinity, single-phase DCPD (CaHPO₄·2H₂O) powders are obtained. Chemical analyses performed on these samples indicate the presence of 1.6 wt% Na and K, combined.

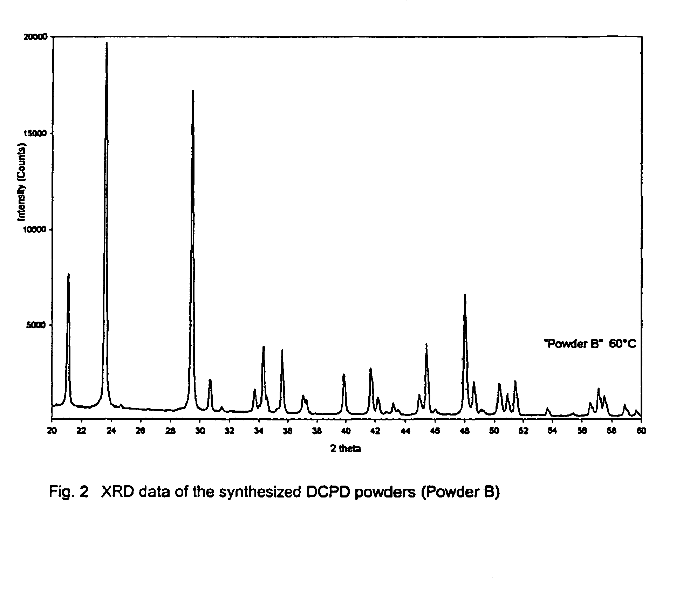

Figure 2 shows the XRD data of the DCPD powders of extremely high crystallinity. These powders have a plate-like morphology (visible by SEM pictures).

### EXAMPLE 3

### Preparation of the cement powders:

Powder A (75 wt%) and Powder B (25 wt%) are placed in a plastic bottle (no grinding balls in it), tightly sealed, and then placed in an automatic mill (Turbula-type) for 1 hour. The total amount of the powder in the bottle is 100 grams. Cement powder is ready after this milling. By mixing these two powders, the phase assemblage of the cement powder corresponded to 71.1 wt% alpha-TCP, 25.2 wt% DCPD, and 3.7 wt% HA, with the overall Ca/P molar ratio being equal to 1.39.

### EXAMPLE 4

### Setting of the cement:

The preferred setting accelerator solution is 3 wt% Na₂HPO₄·2H₂O solution in distilled water. This solution is proven to perform well in alpha-TCP-based cements.

3.00 g Cement powder is first placed into an agate mortar. 1.30 mL of the setting solution is dropped onto the powder body, and the mixture is kneaded with an agate pestle for 90 seconds until the paste was formed. Hardening is observed in 10 to 12 minutes, meaning that before the reaching of the 10 minutes limit, the paste can be given any shape. The compressive strength is measured as 37 ± 2 MPa.

The strength of this cement can be increased up to 50 ± 3 MPa after 15 wt% beta-TCP whisker addition (synthesized in accordance with the procedure outlined in the reference: A.C. Tas, "Molten salt synthesis of calcium hydroxyapatite whiskers, "J. Am. Ceram. Soc., 84, 295-300, 2001) However, such additions do alter the overall Ca/P molar ratio of the original cement formulation.

Compressive strength values are measured in an Instron-tester after squeezing the pastes into 7.5 mm diameter, 1.4 cm tall cylindrical molds, followed by 72 hours of curing at 37°C in deionized water, and drying.

### EXAMPLE 5

### In vitro performance evaluation of the cement:

3.0 g of the cement powder is kneaded in an agate mortar with 1.3 mL of 3 wt% Na₂HPO₄·2H₂O solution for 90 seconds. Formed paste is given the shape of a 1 cm-diameter ball by hand. The samples are then placed in 30 mL of deionized water in sealed glass bottles and placed in an oven at 37°C for periods ranging from 1 day to 3 months.

Scanning electron microscope (SEM) pictures show that the large plates of DCPD already started to transform into calcium-deficient hyroxyapatite (CDHA), whose characteristic morphology is those microflakes or needles. The major component of this cement, which is alpha-TCP (95%) + HA (5%), has also started to transform into CDHA, as evidenced by those microflakes.

SEM pictures which show the morphology of the cement bulk after 3 months in H₂O at 37° C are characterized by an allmost complete dissolution of the plates, leaving a porous cement body, which shall be most suitable for the bone ingrowth to take place and proceed through.

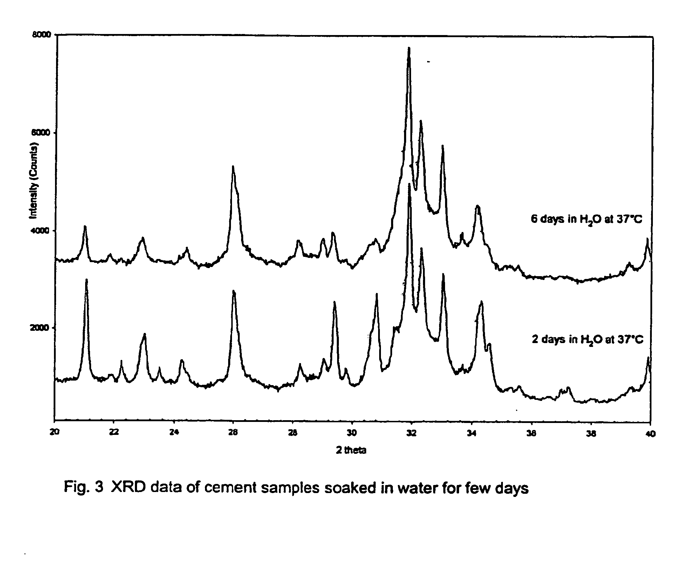

Phase analyses of the cement samples soaked in water at 37°C are reported by the powder XRD data given in Figure 3. It is apparent from this data that CDHA peaks (at the 2 theta regions of around 26° and 31.9° and 35°) started to be visible even after two days of soaking, while the characteristic DCPD peaks (at around 21°, 23°, and 29.5°) are losing their intensity in going from 2 days to 6 days, meaning that they are rapidly dissolving, and turning the whole cement body eventually into one of calcium-deficient hydroxyapatite. CDHA is regarded as the only calcium phosphate compound which strongly resembles to the bone mineral.

## Claims

1. A calcium phosphate cement composition, **characterized in that** it consists of
- a biphasic powder A containing α - type calcium tertiary phosphate (Ca₃(PO₄)₂) and hydroxyapatite (Ca₁₀(PO₄)₆(OH)₂; and
- a single phase powder B containing DCPD (CaHPO₄x 2H₂O)
with an overall molar ratio of Ca/P of 1.35 -1.4.

2. The calcium phosphate cement composition of claim 1, **characterized in that** said powder A and said powder B are mixed in a mixing ratio of 70:30 to 80:20 by weight.

3. The calcium phosphate cement composition of claims 1 or 2, **characterized in that** said powder A and said powder B are mixed in a mixing ratio of 75:25 by weight.

4. The calcium phosphate cement composition of claims 1 to 3, **characterized in that** the particle size is less than 40 µm.

5. The calcium phosphate cement composition of claims 1 to 4, **characterized in that** it has a compressive strength from 34 to 39 MPa after hardening.

6. The calcium phosphate cement composition of claims 1 to 5, **characterized in that** it optionally and additionally consists of 15 wt% beta-type calcium tertiary phosphate (Ca₃(PO₄)₂).

7. The calcium phosphate cement composition of claim 6, **characterized in that** said composition has a compressive strength up to 50±3 MPa after hardening.

8. A method of preparing a calcium phosphate cement composition, **characterized in that** the method comprises the steps of:
a) adding a preheated Ca(NO₃)₂ x 4H₂O solution to a (NH₄)₂HPO₄ solution under stirring followed by addition of concentrated NH₄OH solution and subsequently calcining at about 1200°C of 95 wt% β-type calcium tertiary phosphate and 5 wt% hydroxyapatite to form biphasic powder A consisting of 95 wt% α-type calcium tertiary phosphate and 5 wt% hydroxyapatite,
b) adding a Na₂HPO₄x2H₂O solution to a KH₂PO₄ solution under stirring followed by adding of Ca(NO₃)₂x4H₂O to form single- phase powder B (CaHPO₄x2H₂O); and
c) mixing of powder A with powder B in a mill, and
d) subsequently adding a setting solution (Na₂HPO₄x2H₂O) to form the cement paste with an overall molar ratio of Ca/P of 1.35 - 1.40 and kneading the mixture.

9. The method of claim 8, **characterized in that** powder A and powder B are mixed in a mixing ratio of 70:30 to 80:20 by weight.

10. The method of claim 8, **characterized in that** powder A and powder B are mixed in a mixing ratio of 75:25 by weight.

11. The method of claim 8 to 10, **characterized in that** the setting solution has a concentration of 3 wt%.

12. The method of claim 8 to 11, **characterized in that** the particle size of the calcium phosphate cement composition is less than 40 µm.

13. The method of claim 8 to 12, **characterized in that** it is added 15 wt% β-type calcium tertiary phosphate whisker to increase the strength of the cement up to 50 ± 3 MPa.

## Patentansprüche

1. Calciumphosphatzementzusammensetzung, **dadurch gekennzeichnet, dass** sie aus
- einem zweiphasigen Pulver A, das tertiäres Calciumphosphat (Ca₃(PO₄)₂) vom α-Typ und Hydroxyapatit (Ca₁₀(PO₄)₆(OH)₂) enthält; und
- einem einphasigen Pulver B, das DCPD (CaHPO₄·2H₂O) enthält;
mit einem Gesamtmolverhältnis von Ca/P von 1,35 - 1,4 besteht.

2. Calciumphosphatzementzusammensetzung nach Anspruch 1, **dadurch gekennzeichnet, dass** das Pulver A und das Pulver B in einem Mischungsverhältnis von 70:30 bis 80:20, bezogen auf das Gewicht, gemischt sind.

3. Calciumphosphatzementzusammensetzung nach Anspruch 1 oder 2, **dadurch gekennzeichnet, dass** das Pulver A und das Pulver B in einem Mischungsverhältnis von 75:25, bezogen auf das Gewicht, gemischt sind.

4. Calciumphosphatzementzusammensetzung nach den Ansprüchen 1 bis 3, **dadurch gekennzeichnet, dass** die Teilchengröße weniger als 40 µm beträgt.

5. Calciumphosphatzementzusammensetzung nach den Ansprüchen 1 bis 4, **dadurch gekennzeichnet, dass** sie nach Härtung eine Druckfestigkeit von 34 bis 39 MPa aufweist.

6. Calciumphosphatzementzusammensetzung nach den Ansprüchen 1 bis 5, **dadurch gekennzeichnet, dass** sie gegebenenfalls und zusätzlich aus 15 Gew.-% tertiärem Calciumphosphat (Ca₃(PO₄)₂) vom β-Typ besteht.

7. Calciumphosphatzementzusammensetzung nach Anspruch 6, **dadurch gekennzeichnet, dass** sie nach Härtung eine Druckfestigkeit bis zu 50±3 MPa aufweist.

8. Verfahren zur Herstellung einer Calciumphosphatzementzusammensetzung, **dadurch gekennzeichnet, dass** es die folgenden Schritte umfasst:
a) Zugabe einer vorerhitzten Lösung von Ca(NO₃)₂·4H₂O zu einer Lösung von (NH₄)₂HPO₄ unter Rühren mit nachfolgender Zugabe von konzentrierter NH₄OH-Lösung und anschließende Calcinierung von 95 Gew.-% tertiärem Calciumphosphat vom β-typ und 5 Gew.-% Hydroxyapatit bei etwa 1200°C zur Bildung von zweiphasigem Pulver A, das aus 95 Gew.-% tertiärem Calciumphosphat vom α-Typ und 5 Gew.-% Hydroxyapatit besteht,
b) Zugabe einer Lösung von Na₂HPO₄·2H₂O zu einer KH₂PO₄-Lösung unter Rühren mit nachfolgender Zugabe von Ca(NO₃)₂·4H₂O zur Bildung von einphasigem Pulver B (CaHPO₄·2H₂O) und
c) Mischen von Pulver A mit Pulver B in einer Mühle und
d) anschließende Zugabe einer Abbindelösung (Na₂HPO₄·2H₂O) zur Bildung des Zementleims mit einem Gesamtmolverhältnis von Ca/P von 1,35 - 1,40 und Kneten der Mischung.

9. Verfahren nach Anspruch 8, **dadurch gekennzeichnet, dass** man das Pulver A und das Pulver B in einem Mischungsverhältnis von 70:30 bis 80:20, bezogen auf das Gewicht, mischt.

10. Verfahren nach Anspruch 8, **dadurch gekennzeichnet, dass** man das Pulver A und das Pulver B in einem Mischungsverhältnis von 75:25, bezogen auf das Gewicht, mischt.

11. Verfahren nach Anspruch 8 bis 10, **dadurch gekennzeichnet, dass** die Abbindelösung eine Konzentration von 3 Gew.-% aufweist.

12. Verfahren nach Anspruch 8 bis 11, **dadurch gekennzeichnet, dass** die Teilchengröße der Calciumphosphatzementzusammensetzung weniger als 40 µm beträgt.

13. Verfahren nach Anspruch 8 bis 12, **dadurch gekennzeichnet, dass** man zur Erhöhung der Festigkeit des Zements auf bis zu 50 ± 3 MPa 15 Gew.-% Whisker aus tertiärem Calciumphosphat vom β-Typ zugibt.

## Revendications

1. Composition de ciment au phosphate de calcium, **caractérisée en ce qu'**elle est constituée de
- un poudre à deux phases A contenant du phosphate de calcium tertiaire de type α (Ca₃(PO₄)₂) et de l'hydroxyapatite (Ca₁₀(PO₄)₆(OH)₂) ; et
- une poudre à une seule phase B contenant du DCPD (CaHPO4·2H2O)
présentant un rapport molaire Ca/P total de 1,35 - 1,4.

2. Composition de ciment au phosphate de calcium selon la revendication 1, **caractérisée en ce que** ladite poudre A et ladite poudre B sont mélangées dans un rapport de mélange de 70:30 à 80:20 en poids.

3. Composition de ciment au phosphate de calcium selon les revendications 1 ou 2, **caractérisée en ce que** ladite poudre A et ladite poudre B sont mélangées dans un rapport de mélange de 75:25 en poids.

4. Composition de ciment au phosphate de calcium selon les revendications 1 à 3, **caractérisée en ce que** la granulométrie est inférieure à 40 µm.

5. Composition de ciment au phosphate de calcium selon les revendications 1 à 4, **caractérisée en ce qu'**elle possède une résistance à la compression de 34 à 39 MPa après durcissement.

6. Composition de ciment au phosphate de calcium selon les revendications 1 à 5, **caractérisée en ce qu'**elle est éventuellement constituée en plus de 15 % en poids de phosphate de calcium tertiaire de type β (Ca₃(PO₄)₂).

7. Composition de ciment au phosphate de calcium selon la revendication 6, **caractérisée en ce que** ladite composition possède une résistance à la compression allant jusqu'à 50 ± 3 MPa après durcissement.

8. Procédé de préparation d'une composition de ciment au phosphate de calcium, **caractérisé en ce que** le procédé comprend les étapes consistant à :
a) ajouter une solution de Ca(NO₃)2·4H₂O préchauffée à une solution de (NH₄)₂HPO₄ sous agitation, puis ajouter une solution concentrée de NH₄OH et effectuer ensuite une calcination à environ 1200°C de 95 % en poids de phosphate de calcium tertiaire de type β et 5 % en poids d'hydroxyapatite pour former une poudre à deux phases A constituée de 95 % en poids de phosphate de calcium tertiaire de type α et de 5 % en poids d'hydroxyapatite,
b) ajouter une solution de Na₂HPO₄·2H₂O à une solution de KH₂PO₄ sous agitation, puis ajouter Ca(NO₃)2·4H₂O pour former une poudre à une seule phase B (CaHPO₄·2H₂O) ; et
c) mélanger la poudre A avec la poudre B dans un broyeur, et
d) ajouter ensuite une solution de prise (Na₂HPO₄· 2H₂O) pour former la pâte de ciment présentant un rapport molaire Ca/P total de 1,35 - 1,40 et pétrir le mélange.

9. Procédé selon la revendication 8, **caractérisé en ce que** la poudre A et la poudre B sont mélangées dans un rapport de mélange de 70:30 à 80:20 en poids.

10. Procédé selon la revendication 8, **caractérisé en ce que** la poudre A et la poudre B sont mélangées dans un rapport de mélange de 75:25 en poids.

11. Procédé selon les revendications 8 à 10, **caractérisé en ce que** la solution de prise a une concentration de 3 % en poids.

12. Procédé selon les revendications 8 à 11, **caractérisé en ce que** la granulométrie de la composition de ciment au phosphate de calcium est inférieure à 40 µm.

13. Procédé selon les revendications 8 à 12, **caractérisé en ce que** l'on ajoute 15 % en poids de whisker de phosphate de calcium tertiaire de type β pour augmenter la résistance du ciment jusqu'à 50 ± 3 MPa.
